# EUROPEAN PATENT SPECIFICATION

(11) **EP 3 430 997 B1**
(45) Date of publication and mention of the grant of the patent: **01.11.2023**
(21) Application number: 17765600.6
(22) Date of filing: 17.03.2017
(51) Int. Cl.: A61B 5/378

(54) **MODULAR APPARATUS AND METHOD FOR THE ANALOGOUS SYNCHRONISATION OF ELECTROENCEPHALOGRAMS WITH LUMINOUS EVENTS, OSCILLATORY EVENTS OF ELECTRICAL NATURE, AND MOTOR BEHAVIOUR EVENTS**
MODULARE VORRICHTUNG UND VERFAHREN ZUR ANALOGEN SYNCHRONISATION VON ELEKTROENZEPHALOGRAMMEN MIT LEUCHTENDEN EREIGNISSEN, OSZILLIERENDEN EREIGNISSEN ELEKTRISCHER NATUR UND MOTORVERHALTENSEREIGNISSEN
APPAREIL MODULAIRE ET PROCÉDÉ DE SYNCHRONISATION ANALOGIQUE D'ÉLECTROENCÉPHALOGRAPHIE AVEC DES ÉVÉNEMENTS LUMINEUX, OSCILLATOIRES DE NATURE ÉLECTRIQUE, ET COMPORTEMENTAUX MOTEURS

(30) Priority: 18.03.2016 BR 102016060109
(43) Date of publication of application: 23.01.2019
(73) Proprietor: Fundação Oswaldo Cruz, 21045-900 Rio de Janeiro, RJ (BR)
(72) Inventor: ABRAMOV, Dimitri, Marques, 20261-120 Rio de Janeiro - RJ (BR); GALHANONE, Paulo, Ricardo, 21930-290 Rio de Janeiro - RJ (BR); LIMA, Marcos, Antonio, Dias, 23050-070 Rio de Janeiro - RJ (BR); MARTINS, Carlos, Henrique, Quintanilha, 20521-050 Rio de Janeiro - RJ (BR)
(74) Representative: Lavoix
(86) International application number: PCT/BR2017/050062
(87) International publication number: WO 2017/156613

(56) References cited:
- US-A- 3 498 287
- US-A- 4 195 626
- US-A- 4 913 160
- US-A- 5 447 166
- US-A1- 2005 177 065
- US-A1- 2010 145 215
- US-A1- 2010 280 403
- US-A1- 2014 058 241
- US-A1- 2015 126 802
- US-B2- 8 938 289

## Description

### FIELD OF THE INVENTION

The device constituting this invention is intended to synchronize electroencephalograms (EEGs), recorded by any digital equipment, with physical events (sensorial, visual or auditory stimuli) and behavioral events (motor response, vocal response), thus allowing EEG signal mediation for the purpose of visualizing Evoked Potentials or Event-Related Potentials, which are important research subjects in the neurosciences and clinical investigations into neurological and psychiatric pathologies. This invention allows any digital EEG device to be transformed into equipment that can record Evoked Potentials. The invention also provides a method for the analog synchronization of EEGs with oscillating electric light-related events and motor behaviors.

### BACKGROUND OF THE INVENTION

Evoked Potentials (PE) are electrophysiological responses evoked from the nervous system by a variety of events, such as endogenous cognitive processes or sensorial stimuli synchronized with a behavioral response. There are currently two alternative methodologies for materializing Evoked Potentials:
- using dedicated equipment for materializing Evoked Potentials (such as that made by the Nicolet^{®}, Nihon Kohden^{®} and Neurotec^{®} manufacturers, for example). Despite the high quality of the tests that they produce, they are systems designed to provide medical care and with few stimulation protocols, hampering the customization of tests. Consequently, such equipment is of little use for research purposes. These items of equipment are extremely expensive (they are more costly than the electroencephalogram equipment made by the same manufacturers) and have few channels (average of four). Equipment prices rise steeply for more channels.
- Methodologies using digital electroencephalography equipment linked to computer systems, such as the following, for example:
- Pscychotoolbox-MatLab^{®}, CORTEX^{®} and Presentation^{®}. These computer systems produce stimulation protocols for obtaining Evoked Potentials, in parallel to a programmable trigger, synchronized with the events in these protocols, which is sent to the EEG equipment through an outlet port on the computer. These systems are feasible only for scientific research, for the following reasons: high complexity of these systems (for their installation, maintenance, operation and programming) which normally require a technician with professional qualifications, found only in laboratories with ample expertise; possibility of synchronization errors resulting from programming mistakes or even computer problems intrinsic to extremely complex operating systems (such as Windows^{®}) that might adversely affect the accurate timing of software-hardware-EEG interactions. All these variables undermine the feasibility of using this methodology for medical care.

Document US 2005/177065 discloses a system for testing a subject's cognition and motor timing which includes an actuator, a sensor and a computer. The actuator is configured to present to the subject multiple stimuli, including predictable stimuli and non-predictable (e.g., random or pseudo-random) stimuli.

Document US 5 447 166 discloses a human-computer interface which uses neuroelectric signals recorded from the user's scalp i.e. electroencephalograms (EEGs) to alter the program being run by the computer, for example to present less or more difficult material to the user, depending on the user's neurocognitive on-line workload score.

Document US 2010/280403 discloses a system and a method in which least one sequence of a plurality of stimuli is presented to an individual (using appropriate sensory modalities), and the time course of at least one measurable response to the sequence(s) is used to select at least one stimulus from the sequence(s). The sequence(s) may be dynamically altered based on previously selected stimuli and/or on estimated probability distributions over the stimuli and such dynamic alteration may be based on predictive models of appropriate sequence generation mechanisms, such as an adaptive or static sequence model.

Document US 2014/058241 discloses a monitoring device for monitoring the brain activity of a patient response to one or more stimuli provided to the patient. Data from the monitoring device is analyzed to determine a measure of central nervous system condition for the patient.

Document US 3 498 287 discloses an apparatus and a method employing a stimulative event to trigger responsive EEG signals that are analyzed by zero-crossing detectors to provide a photographic readout from which the subjct's response time to the event may be determined.

Document US 4 195 626 discloses a biofeedback chamber for applying audible, visual, electrical or tactile stimuli to a subject according to a rhythmical pattern under microprocessor control.

There is a need at the state of the art for the development of a definitive and innovative conceptual technology that can handle the materialization of Neurophysiology Event Protocols (Evoked Potentials) and ensuring: accurate synchronization of events and signals; procedures that are easy to perform; low costs; and universal access to Evoked Potential tests by countless electroencephalography units already in place or to be implemented

### BACKGROUND OF THE INVENTION

This purpose of this invention is to provide accurate marking of stimuli presentation instants and responses to these stimuli in Neuroscience and Evoked Potentials research protocols for clinical neurophysiology, with no need for computer systems equipped with stimulation management applications.

This invention proposes a device according to claim 1 and a method according to claim 6 that allow synchronization between sensorial stimuli and an electroencephalogram (EEG) in order to obtain Evoked Potentials.

The device resulting from this invention allows Event-Related Potentials (Evoked Potentials) to be obtained through using ordinary EEG equipment, through the transduction of the events submitted to the subject (or behavioral events) in an analog signal that synchronizes these events with the encephalographic recording, generating trustworthy latencies in the evoked potential wave.

The device resulting from this invention allows **synchronization of the electroencephalogram (EEG)** recorded by any digital equipment or through analog-digital conversion with **events:** (1) **light,** emitted by any source, such as the sun, liquid-crystal display (LCD), cathode-ray tube (CRT), cinematographic projections, all types of lamps and all types of light-emitting diodes (LEDs), in any color (wavelength) within the visible spectrum and at any intensity; (2) **electrical oscillations** at any frequency, voltage and current, deriving from (2.a) from the outlet of an analog or digital electronic sound source (electric signal outlet to loudspeakers, earphones or amplifiers, either monophonic or stereophonic); (2.b) from a transduction device for another physical event into an electrical event (transducer of voltage, vibration, sound, pressure, temperature, humidity, flow or any biological events); (2.c.) From an electric signal conditioning device (amplifiers or transformers), (2.d) from a skin impedance measurement device, with or without reproduction of the respective electric signal to a parallel outlet; and (3) **behavioral motor responses** produced actively in all four limbs, either individually or in pairs, through activating switch devices; producing square electrical envelopment waves (i.e., triggers) whose lengths vary in order to demarcate the event in time, transmitting these triggers to the EEG equipment through its DC channels (at voltages between -5V and +5V) or AC (for example, -50mV a +50 mV), that are recorded on the electroencephalogram concomitantly with the biological signal.

Through the device constituting this invention, it is possible to produce EEG signal mediation for the purpose of visualizing Evoked Potentials or Event-Related Potentials, after the recording thereof, as a subject for neuroscience research and clinical investigations of neurological and psychiatric pathologies. This invention allows any digital EEG device to be transformed into equipment that can record Evoked Potentials.

Among the advantages of this invention, we may mention:
- with markings taken directly from the presented stimuli and the responses generated by patients, delays are fixed and known, defined only by the electronic signal spread times, which can thus be easily offset through software during the post-analysis stage;
- with the analog generation circuit of the trigger, the signal is already defined by being introduced directly into the DC or even AC channels;
- any digital EEG equipment may be used as off-line Evoked Potentials (ERP) equipment, which could make the use of medium and long latency Evoked Potentials more popular in neurological clinical practice, disseminating a low-cost safety technology for functional research into the nervous system (useful for monitoring patients with demyelinating diseases and diagnoses of visual tract lesions, for example); and,
- stimuli management applications are not required. Any software or even a sequence of stimuli recorded in a media file may be used for stimulation

This invention proposes a novel functional concept that lies in the analog synchronization of EEG signals with physical and behavioral events in order to obtain Evoked Potentials through a device linked to the EEG and external to this unit.

The device presented here exemplifies the implementation of the invention in question, as it allows Event-Related Potentials (Evoked Potentials) to be obtained through using ordinary digital EEG equipment by electronic manipulation of the events submitted to the subject (light stimuli transduced by photocell, or electrical oscillations deriving from sound equipment outlets, the transduction of other physical events, conditioning, biological signals or skin impedance measurements related to such events or behavioral events (motor responses of the upper and / or lower limbs) in an analog signal that synchronizes these events with the encephalographic recording, for easier examination of the exact evoked potential wave latencies, as the trigger markings are directly derived from analog processing of the presented stimuli and the responses generated by the patients. The delays inherent to analog processing time are set and known, defined only by the electronic signals spread time, which can thus be easily offset through software in the post-analysis stage. With the analog generation circuit of the trigger, the signal is already defined by being introduced directly into the DC or even AC channels. This means that any digital EEG equipment may be used for off-line processing of evoked potentials. As digital EEG equipment has a relatively low analog-digital conversion time (1000 points per second or less), only medium and long latency evoked potentials (greater than 50 ms) can be analyzed safely with this conventional equipment. We note that this methodology will be appropriate for detecting visual, medium-latency auditory and cognitive evoked potentials (endogenous, related to the motor responses), thus disseminating a safe and low-cost functional technology for examining the central nervous system during research activities as well as medical care (where it is very useful for monitoring patients with demyelinating diseases, visual system alterations evaluations and studies of the progression of cognitive diseases, for example); there is no need for very expensive dedicated equipment and computer systems with stimuli management applications. Any software may be used for stimulation or even a sequence of stimuli recorded in a media file.

### BRIEF DESCRIPTION OF THE FIGURES

- **Figures 1A** and **1B**: offer a general schematic overview of the device constituting the invention in a preferred embodiment.
- **Figures** 2.1, 2.2 and 2.3: offer a schematic view of the necessary elements in the top and back panels of the standard device addressed by the invention, schematized with dashed lines showing the functional divisions of the system into modules.
- **Figure 3**: shows the button unit for the device addressed by the invention.
- **Figure 4**: conceptually demonstrates the function of the invention.
- **Figure 5**: shows the scheme in concept blocks for the functioning of the invention, which can be generalized or any circuit playing this role.
- **Figure 6**: is a representation of the modular device addressed by the invention.

### DETAILED DESCRIPTION OF THE INVENTION

The basic elements of the device addressed by the invention are presented below.

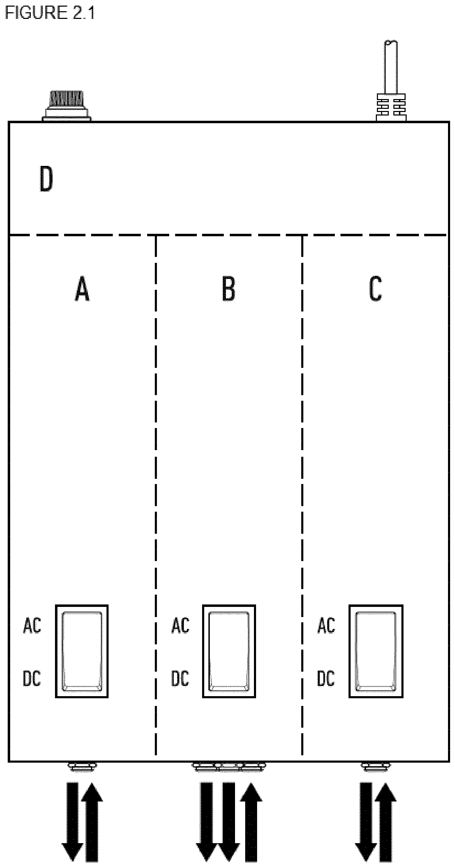

The device constituting the invention is a ***modular item of equipment**,* with three types of function modules: a motor response processing module (A, as shown in **Figure** 2.1); electrical oscillation processing module (B, as shown in **Figure** 2.1); and the light processing module (C, as shown in **Figure** 2.1). Module D is the power feed and control module, containing the power switches, the power input and the voltage safety control (fuse), that distributes electricity to all three (or more) function modules.

A unit of the device constituting the invention may contain as many function modules as wished, in a customized manner. For example, two modules B, one module A and two modules C. As modules A and B have two input channels each, generating trigger waves at the outlet in a single channel, with different amplitude levels, which can indicate whether there was a response or the presence of a stimulus in one, the other or both module input channels.

In order to demonstrate the operations of this invention, a unit was set up for the device (described here) with three function modules (with 5 input channels and three output channels that together produce five individual response patterns), in the belief that much of the demand will be met with this configuration. Nevertheless, other configurations may be set up through arraying the function modules side-by-side on a single level (keeping the thickness of the device), with a single circuit board. Further explanations are presented in greater detail in the references for the **Figure**s.

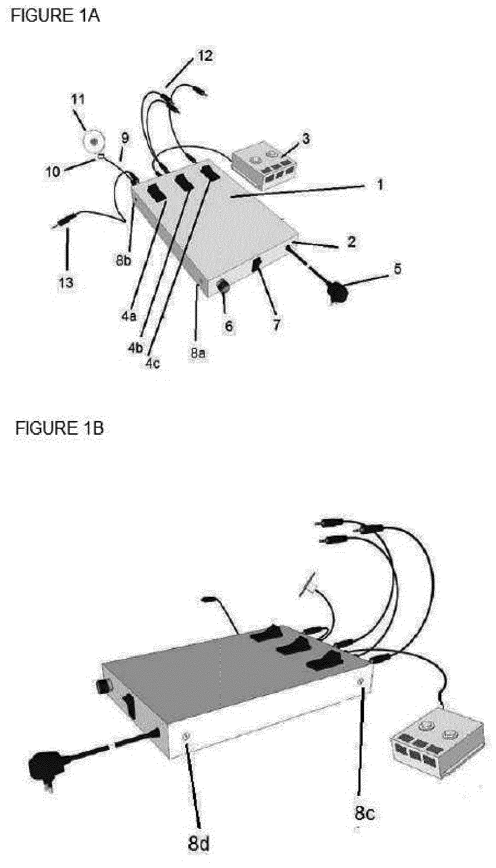

The numerical references in **Figure 1** represent a unit of the device constituting the invention with three modules, one of each type: A, B and C. Representing a preferred mode of the invention, **Figure** 1 presents the following structural elements:
**(1) Housing Lid:** sheet made from metal or plastic material forming the top and sides of the outer housing of the device, with top openings for the 4a-c switches.
**(2) Housing Body:** composed of the outer front, back and lower parts of the housing with all the plugs and outlets of the device, and holding its circuit board. There are screws on the side of the body to hold the housing lid in place. There is a crossbar on the housing body along the length of the device, fixed to its sides, where the 4a-c selection switches are attached.
**(3) Button Unit:** composed of two buttons for entering motor responses.
**(4) a-c** switches that select voltage levels at the outlets for each outlook function module (a - motor response module; b - electrical oscillations module; and c - light detection module). If DC is selected, the output voltage varies between -5V and +5V (for entering the DC channels of the EEG equipment). Is AC is selected, the output voltage varies between -50 and +50mV (for the AC channels of the EEG equipment, or for entering the EEG channel).
**(5) Power Source:** the device uses a regular power source with a switch, with an inflow of alternating current at 100-240V, 50-60Hz, and an outflow of continuous current at no more than 12V and 1000mA.
**(6) Threaded fuse port**
**(7) Power switch.**
**(8) Screws a-d:** for attaching the lid of the device to its body
**(9) Photocell input cable**
**(10) Suction cup:** the photocell is attached to a smooth surface (an LCD or CRT screen, for example) by a suction cup. When necessary, this allows the photocell to be attached to any other type of surface by any other means whatsoever, such as adhesive tape.
**(11) Photocell:** on the device, we use a broad-spectrum visible light energy uptake current-breaking photocell.
**(12) Function module trigger output tables:** these are the cables connecting to the DC and AC channels on the EEG equipment. The standard outlets on the embodied device are P1 mono type. Many types of equipment use different connectors (such as RCA); in this case, adapters (*rabichos*) that are found easily on the market would be needed to use the device constituting the invention.
**(13) Electrical oscillations input table:** set up with a stereo P2 connector, in order to link directly into an audio outlet (speaker out) on many different types of equipment, ranging from computers to television sets, mp3 and DVD players, as the use of this module to synchronize sound stimuli will be the rule. In order to connect other devices, adapters may be needed or even signal adjusters (for electrical oscillations coming from sensors or other measurement devices/biological examinations).

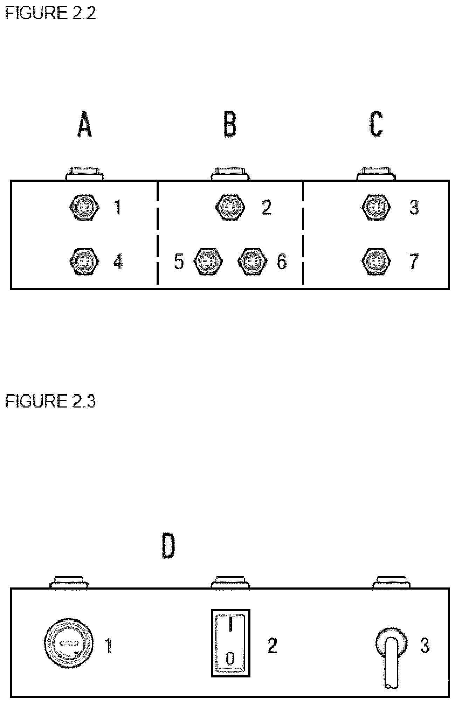

**Figure 2****.****1** shows a schematic view of the top panel of the device addressed by the invention. The dashed lines represent imaginary divisions of the four modules constituting the preferred configuration, with three function modules (A: motor response module, B: electrical oscillations module, C: light detection module), and the power feed module (D), which constitutes the entire front part of the device, as shown in **Figure** 1. It is noted that the switches (corresponding to Switches 4a-c in **Figure** 01) of the respective modules define the output voltage range for each module: in the AC position, the voltage range is compatible with the standards for digital EEG devices (no more than 50mV to +50mV), and for the DC position, the voltage range is -5 a +5V. The arrows represent the number of inlets and outlets for each function module.

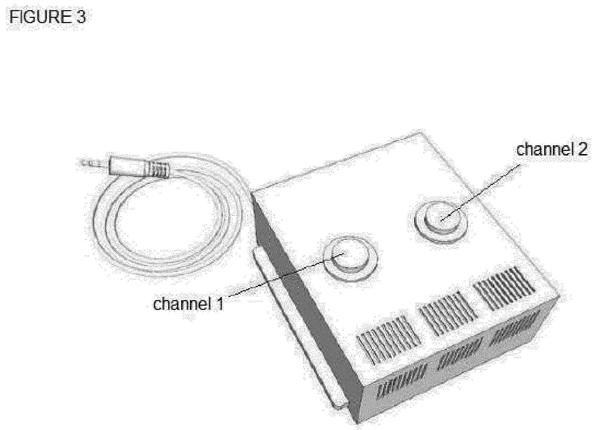

**Figure 2.2** shows a rear view of the device in a preferred embodiment (see **Figure** 2.1), containing the device signal inlets and outlets. All outlets are square waves with durations identical to the input signal, with ranges depending on whether they are intended for a DC or AC channel (defined by the position of Selector Switches 4a to c). Specifically, the elements in the **Figure** are:
**(1) Behavioral response inlet:** two single-reference channels that are connected to the button unit (see **Figure** 3) by a stereo connector (2 phases, 1 neutral).
**(2) Electrical oscillations inlet:** two single-reference input channels with a stereo connector (2 phases, 1 neutral), that detects oscillations at less than 100 Hz as individual events, and oscillations at more than 100Hz at a frequency compatible with most soundwaves, as a single event.
**(3) Photocell inlet**
**(4) Motor response module trigger outlet:** Pressing Button 1 (channel 1) produces a positive wave of +3V (+30mV if the AC option is selected on corresponding Switch 4 - see **Figure** 1); pressing Button 2 (channel 2) produces a positive wave of +2V (+20mV if the AC option is selected); while pressing both buttons simultaneously produces a positive wave of +5V(+50mV if the AC option is selected)
**(5) Electrical oscillation module trigger outlet:** detecting electrical oscillations events produces 2, 3 or 5V waves (20, 30 or 50mV for the respective AC options), for either the right, the left or both input channels during the event, which has a set response latency (measured between the peak of the first oscillation and maximum trigger voltage) of 4 mseg (four oscillations lasting 10 mseg or more), or 3 mseg (four oscillations lasting less than 10 mseg).
**(6) Electrical oscillation outlet:** a female stereo connector that reproduces the respective input signal from both channels. This is useful for signal sharing with another device or to activate a speaker (loudspeakers, earphones, etc).
**(7) Light module trigger outlet:** the light module produces a 5V trigger wave (50mV for the AC option), with latency set for a maximum voltage of 2mseg. On the threshold of inactivity, the maximum detection frequency for light pulses is 30Hz or more. For the xenon lamp flashes (color 6500 K, 30 cd/m²/W, pulse duration: 20 ps), the maximum detection frequency is 60Hz or more. For an LCD or CRT display operating at 60Hz (our standard), the maximum stimulation frequency is 30 Hz (one light cycle, one dark cycle). See **Figure** 8.

**Figure 2****.****3** provides a front view of the back of the device in a preferred embodiment, which has the controls and the module D input. Specifically, the elements in this Figure are:
(1) Threaded fuse port.
(2) Power switch.
(3) Power cable input: from the power source, (see **Figure** 1)

**Figure 3** shows a preferred button unit fitted to the device addressed by the invention. Each button is a channel (channel 1, left button; channel 2, right button). Note the output cable with a two-channel connector (stereo). The preferred button unit is lightweight (under 0.5 kg), with large buttons that are well adapted to use by the hands and big toes. A broader variation of this button unit may be implemented for behavioral responses with the feet.

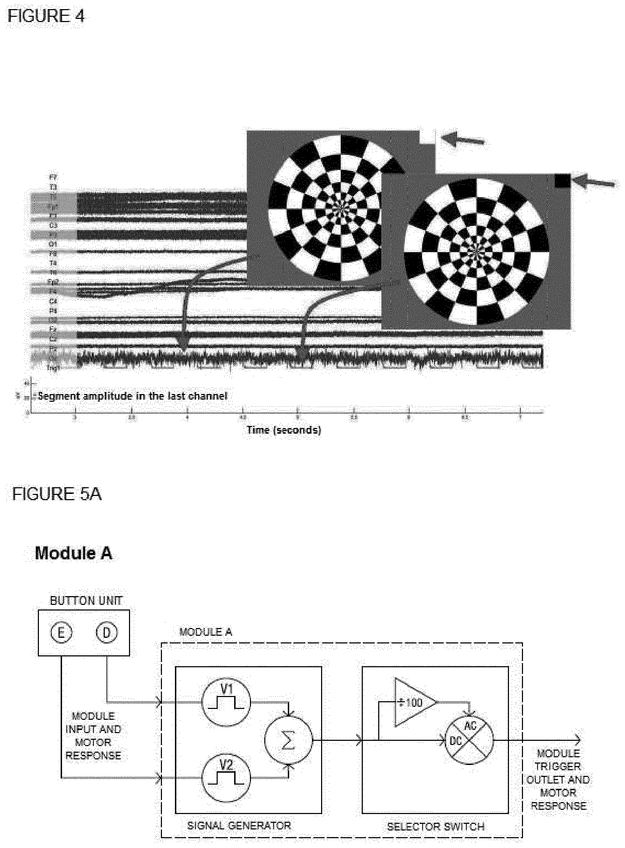

**Figure 4** presents an illustration of the functional paradigms of the device addressed by this invention, for a general understanding of the functioning of the device. Using any application (software) or even a media file played on a media player (such as audio files or video files in formats such as *avi or *mp4, as shown in this Figure), the stimulation pattern is presented to the subject whose evoked potentials are being evaluated. With the stimulation pattern, a small area along the edges alternate between white and black (presence and absence of light, indicated by the straight arrow) depending on light reversals in the target pattern fields, synchronizing the alternation of these patterns through the light detection module. On the electroencephalogram, the device constituting this invention "marks" the moment of each reversal (see the curved arrows), in order to allow studies of the evoked potentials in terms of the visual stimulation provided by this reversed pattern. The dotted line with the square waves represents the figure signal, sent from the device through the DC channel (through which the trigger signal from the light detection model enters the EEG equipment), overlaid on the electroencephalogram. The unbroken line is the EEG readings.

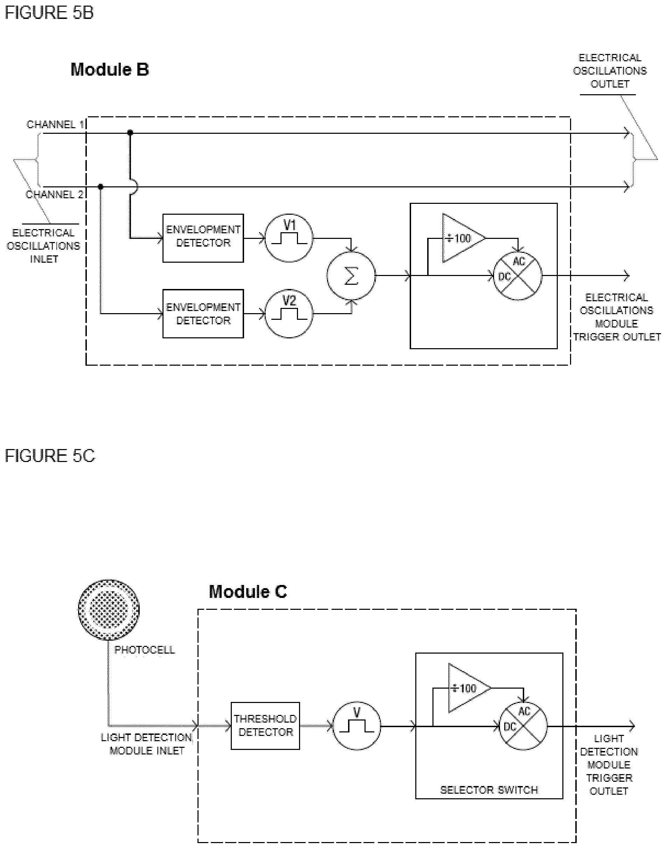

**Figure 5****:** presents the function block diagram of the invention:
Figure 5.A: describes the functioning of the motor response processing module. Pressing the right and/or left buttons on the button unit triggers the V1 and/or V2 voltage signal generator that, depending on the selector switch position, transmits in the form of square waves (with the same duration as the button is pressed) at voltages compatible with the DC or AC channels (÷ 100).

**Figure** 5-B: describes the functioning of the electrical oscillation processing module. The electrical signals that enter through channel 1 and / or channel 2 runs through enveloping detector that generates a V1 and / or V2 voltage signal that, depending on the selector switch position, transmits in the form of square waves (with the same duration as the electrical oscillations in the input channels) at voltages compatible with the DC or AC channels (÷ 100). In parallel, there is a repeat system sending signals in channel 1 and 2 to an electrical oscillation output.

**Figure** 5-C: describes the functioning of the light processing module. The electrical signal from the photocell runs through the threshold detector that generates a V1 voltage signal that, depending on the selector switch position, transmits in the form of square waves (with the same duration as the light signal activating the photocell) at voltages compatible with the DC or AC channels (÷ 100).

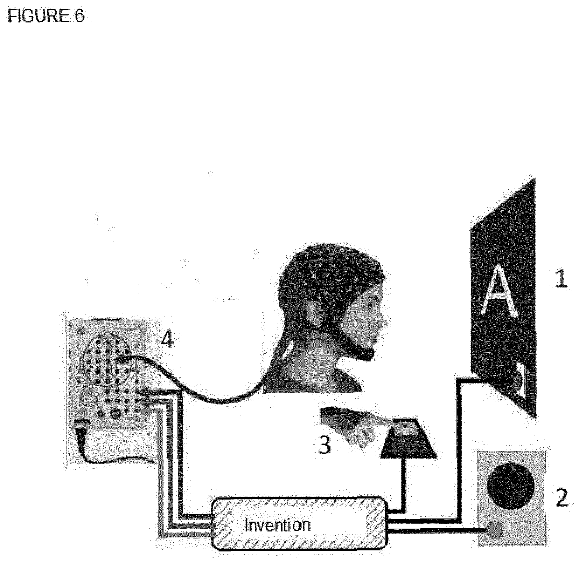

**Figure 6** shows a representation with three modules of the modular device constituting the invention, in a preferred presentation, with one motor response processing module (3), one electrical oscillation processing module (2), and one light processing module (1). In the context of this Figure, we present a schematic diagram of the application of the device constituting the invention during the examination of a subject completing a test in which pictures are displayed (synchronized with the EEG [4] through the light processing module) and sounds (synchronized with the EEG through the electrical oscillation processing module), with the motor responses collected (and synchronized with the EEG) through the motor response processing module.

Consequently, so the device described here, it is possible to define and record markings taken directly from the presented stimuli and the responses generated by patients through only the electronic signal spread time.

Although the invention is described in details and refers to specific examples thereof, it will be clear to any specialist in this field that many changes and modifications may be made without moving away from its scope.

## Claims

1. Modular device for analog electroencephalography synchronization with oscillating electrical light-related events and motor behaviors, comprising function modules of the following types:
a motor response processing module (A);
an electrical oscillation processing module (B);
a light processing module (C); and
a power feed and control module (D) comprising a power switch (7), a power input and a voltage safety control, and means to distribute electricity to the function modules;
wherein the motor response processing module (A) comprises a selector switch (4a), a behavioral response inlet comprising two single-reference channels connectable to a button unit (3) and a motor response module trigger outlet, wherein the motor response processing module (A) is configured to produce at its outlet square waves with durations substantially identical to the signal at the inlet, wherein the voltage range of said square waves depends on the position of the selector switch (4a) and is intended for a DC or AC channel of digital EEG equipment; and
wherein the electrical oscillation processing module (B) comprises a selector switch (4b), an electrical oscillations inlet comprising two single-reference input channels and an electrical oscillation module trigger outlet, wherein the electrical oscillation processing module (B) is configured to produce at its outlet square waves with durations substantially identical to the signal at the inlet, wherein the voltage range of said square waves depends on the position of the selector switch (4b) and is intended for a DC or AC channel of digital EEG equipment; and
wherein the light processing module (C) comprises a selector switch (4c), a photocell inlet (3), and an electrical oscillation module trigger outlet, wherein the light processing module (C) is configured to produce at its outlet square waves with durations substantially identical to the signal at the inlet, wherein the voltage range of said square waves depends on the position of the selector switch (4c) and is intended for a DC or AC channel of digital EEG equipment.

2. Device according to Claim 1, wherein it contains one or more function modules as desired selected from the group consisting of the light processing module (C), the electrical oscillation processing module (B), and the motor response processing module (A).

3. Device according to Claim 1, wherein the two single-reference channels of the behavioral response inlet of the motor response processing module (A) are configured to be connected to the button unit (3) by a stereo connector.

4. Device according to Claim 1, wherein the electrical oscillations inlet of the electrical oscillation processing module (B) is configured to be compatible with standard audio channel's output to link directly into an audio outlet.

5. Device according to Claim 4, wherein the electrical oscillations inlet of the electrical oscillation processing module (B) is configured for connecting specific transducers for physical events, such as temperatures, pressures or magnetic fields, and also for physiological events such as muscle activity, strength, skin conductance or ECG.

6. Method for analog synchronization of electroencephalography with oscillating electrical light-related events and motor behaviors, comprising the following steps:
uptake of physical light-type events, electrical oscillations and similar and/or behavioral type motor responses from a subject;
generating square wave trigger signal with substantially the same duration as the recorded events using the device according to claim 1; and
transmission of the trigger signal to a digital electroencephalography equipment through AC or DC channels.

## Patentansprüche

1. Modulares Gerät zur analogen Elektroenzephalographie-Synchronisation mit oszillierenden, elektrischen, lichtbezogenen Ereignissen und motorischen Verhaltensweisen, umfassend Funktionsmodule der folgenden Typen:
ein Modul zur Verarbeitung motorischer Reaktionen (A);
ein Modul zur Verarbeitung elektrischer Schwingungen (B);
ein Lichtverarbeitungsmodul (C); und
ein Stromzufuhr- und Steuermodul (D), das einen Stromschalter (7), einen Stromeingang und eine Spannungssicherheitssteuerung sowie Mittel zum Verteilen von Strom an die Funktionsmodule umfasst;
wobei das Modul zur Verarbeitung motorischer Reaktionen (A) einen Wahlschalter (4a), einen Eingang für verhaltensbezogene Reaktionen, der zwei Einzelreferenzkanäle umfasst, die mit einer Tasteneinheit (3) verbunden werden können, und einen Auslöserausgang für das Modul zur Verarbeitung motorischer Reaktionen umfasst, wobei das Modul zur Verarbeitung motorischer Reaktionen (A) so konfiguriert ist, dass es an seinem Ausgang Rechteckwellen mit Laufzeiten erzeugt, die im Wesentlichen mit dem Signal am Eingang identisch sind, wobei der Spannungsbereich dieser Rechteckwellen von der Position des Wahlschalters (4a) abhängt und für einen Gleich- oder Wechselstromkanal einer digitalen EEG-Ausrüstung bestimmt ist; und
wobei das Modul zur Verarbeitung elektrischer Schwingungen (B) einen Wahlschalter (4b), einen Eingang für elektrische Schwingungen, der zwei Einzelreferenz-Eingangskanäle umfasst, und einen Auslöserausgang für das Modul zur Verarbeitung von elektrischen Schwingungen umfasst, wobei das Modul zur Verarbeitung von elektrischen Schwingungen (B) so konfiguriert ist, dass es an seinem Ausgang Rechteckwellen mit einer Laufzeit erzeugt, die im Wesentlichen mit dem Signal am Eingang identisch ist, wobei der Spannungsbereich dieser Rechteckwellen von der Position des Wahlschalters (4b) abhängt und für einen Gleich- oder Wechselstromkanal einer digitalen EEG-Ausrüstung bestimmt ist; und
wobei das Lichtverarbeitungsmodul (C) einen Wahlschalter (4c), einen Fotozelleneingang (3) und einen Auslöserausgang für ein Modul für elektrische Schwingungen umfasst, wobei das Lichtverarbeitungsmodul (C) so konfiguriert ist, dass es an seinem Ausgang Rechteckwellen mit einer Laufzeit erzeugt, die im Wesentlichen mit dem Signal am Eingang identisch ist, wobei der Spannungsbereich dieser Rechteckwellen von der Position des Wahlschalters (4c) abhängt und für einen Gleich-oder Wechselstromkanal einer digitalen EEG-Ausrüstung bestimmt ist.

2. Vorrichtung nach Anspruch 1, wobei sie wahlweise ein oder mehrere Funktionsmodule enthält, ausgewählt aus der Gruppe bestehend aus dem Lichtverarbeitungsmodul (C), dem Modul zur Verarbeitung elektrischer Schwingungen (B) und dem Modul zur Verarbeitung motorischer Reaktionen (A) enthält.

3. Vorrichtung nach Anspruch 1, wobei die beiden Einzelreferenzkanäle des Eingangs für verhaltensbezogene Reaktionen des Moduls zur Verarbeitung motorischer Reaktionen (A) so konfiguriert sind, dass sie über einen Stereoanschluss mit der Tasteneinheit (3) verbunden sind.

4. Vorrichtung nach Anspruch 1, wobei der Eingang für elektrische Schwingungen des Moduls zur Verarbeitung elektrischer Schwingungen (B) so konfiguriert ist, dass er mit dem Ausgang eines Standard-Audiokanals kompatibel ist, damit er direkt mit einem Audioausgang verbunden werden kann.

5. Vorrichtung nach Anspruch 4, wobei der Eingang für elektrische Schwingungen des Moduls zur Verarbeitung elektrischer Schwingungen (B) für den Anschluss spezifischer Wandler für physikalische Ereignisse, wie Temperaturen, Drücke oder Magnetfelder, und auch für physiologische Ereignisse, wie Muskelaktivität, Kraft, Hautleitwert oder EKG, konfiguriert ist.

6. Verfahren zur analogen Synchronisation von Elektroenzephalographie mit oszillierenden, elektrischen, lichtbezogenen Ereignissen und motorischen Verhaltensweisen, umfassend die folgenden Schritte:
Aufnehmen physikalischer lichtartiger Ereignisse, elektrischer Schwingungen und ähnlicher und/oder verhaltensbezogene Motorreaktionen einer Versuchsperson;
Erzeugen eines Rechteckwellen-Auslösesignals mit im Wesentlichen der gleichen Laufzeit wie die aufgezeichneten Ereignisse unter Verwendung der Vorrichtung nach Anspruch 1; und
Übertragen des Auslösesignals an ein digitales Elektroenzephalographiegerät über Wechsel- oder Gleichstromkanäle.

## Revendications

1. Dispositif modulaire pour la synchronisation de l'électroencéphalographie analogique avec des événements électriques oscillants liés à la lumière et des comportements moteurs, comprenant des modules fonctionnels des types suivants :
un module de traitement de la réponse motrice (A) ;
un module de traitement des oscillations électriques (B) ;
un module de traitement de la lumière (C) ; et
un module d'alimentation et de commande (D) comprenant un interrupteur de puissance (7), une entrée de puissance et un contrôle de sécurité de la tension, et des moyens de distribution de l'électricité aux modules fonctionnels ;
dans lequel le module de traitement de la réponse motrice (A) comprend un sélecteur (4a), une entrée de réponse comportementale comprenant deux canaux à référence unique pouvant être connectés à une unité à boutons (3) et une sortie de déclenchement du module de réponse motrice, dans lequel le module de traitement de la réponse motrice (A) est configuré pour produire à sa sortie des ondes carrées avec des durées sensiblement identiques au signal à l'entrée, dans lequel la plage de tension desdites ondes carrées dépend de la position du sélecteur (4a) et est destinée à un canal CC ou CA d'un équipement EEG numérique ; et
dans lequel le module de traitement des oscillations électriques (B) comprend un sélecteur (4b), une entrée d'oscillations électriques comprenant deux canaux d'entrée à référence unique et une sortie de déclenchement du module d'oscillations électriques, dans lequel le module de traitement des oscillations électriques (B) est configuré pour produire à sa sortie des ondes carrées avec des durées sensiblement identiques au signal à l'entrée, dans lequel la plage de tension desdites ondes carrées dépend de la position du sélecteur (4b) et est destinée à un canal CC ou CA d'un équipement EEG numérique ; et
le module de traitement de la lumière (C) comprend un sélecteur (4c), une entrée de cellule photoélectrique (3) et une sortie de déclenchement de module d'oscillation électrique, le module de traitement de la lumière (C) étant configuré pour produire à sa sortie des ondes carrées d'une durée sensiblement identique à celle du signal à l'entrée, dans lequel la plage de tension desdites ondes carrées dépend de la position du sélecteur (4c) et est destinée à un canal de courant continu ou alternatif d'un équipement d'EEG numérique.

2. Dispositif selon la revendication 1, dans lequel il contient un ou plusieurs modules fonctionnels au choix choisis dans le groupe constitué du module de traitement de la lumière (C), du module de traitement des oscillations électriques (B) et du module de traitement de la réponse motrice (A).

3. Dispositif selon la revendication 1, dans lequel les deux canaux à référence unique de l'entrée de la réponse comportementale du module de traitement de la réponse motrice (A) sont configurés pour être connectés à l'unité à boutons (3) par un connecteur stéréo.

4. Dispositif selon la revendication 1, dans lequel l'entrée des oscillations électriques du module de traitement des oscillations électriques (B) est configurée pour être compatible avec la sortie d'un canal audio standard afin d'être reliée directement à une prise audio.

5. Dispositif selon la revendication 4, dans lequel l'entrée des oscillations électriques du module de traitement des oscillations électriques (B) est configurée pour connecter des transducteurs spécifiques pour des événements physiques, tels que des températures, des pressions ou des champs magnétiques, et également pour des événements physiologiques tels que l'activité musculaire, la force, la conductance de la peau ou l'ECG.

6. Procédé de synchronisation analogique de l'électroencéphalographie avec des événements électriques oscillants liés à la lumière et des comportements moteurs, comprenant les étapes suivantes :
l'absorption d'événements physiques de type lumineux, d'oscillations électriques et de réponses motrices similaires et/ou de type comportemental de la part d'un sujet ;
la génération d'un signal de déclenchement à ondes carrées d'une durée sensiblement identique à celle des événements enregistrés en utilisant le dispositif selon la revendication 1 ; et
la transmission du signal de déclenchement à un équipement d'électroencéphalographie numérique par des canaux à courant alternatif ou continu.
